# EUROPEAN PATENT APPLICATION

(11) **EP 4 825 308 A1**
(43) Date of publication of application: **21.10.2026**
(21) Application number: 25170293.2
(22) Date of filing: 14.04.2025
(51) Int. Cl.: A61B 5/00, A61B 5/024, A61B 5/1455, A61B 5/08

(54) **OPTICAL MEASUREMENT DEVICE**

(71) Applicant: Koninklijke Philips N.V., 5656 AE Eindhoven (NL)
(72) Inventor: PAULUSSEN, Elvira Johanna Maria, 5656 AG Eindhoven (NL); KOSTAKIS, Dimitri George, 5656 AG Eindhoven (NL)
(74) Representative: Philips Intellectual Property & Standards

(57) **Abstract**

An optical measurement device (100) for obtaining optical measurements from a subject sampling region (110), is provided. The optical measurement device includes: at least one optical source (120, 120') configured to generate optical radiation; an optical waveguide (130); an optical splitter (140) or circulator (150); and at least one optical detector (160). The at least one optical source (120, 120') is configured to couple the optical radiation into a proximal end of the optical waveguide (130). The optical splitter (140) or circulator (140) is arranged at a distal end of the optical waveguide (130) and is configured to output at least a portion of the optical radiation for irradiating the subject sampling region (110), and to receive at least a portion of the outputted optical radiation that has irradiated the subject sampling region, and to couple at least a portion of the received portion back into the distal end of the optical waveguide (130). The at least one optical detector (160) is arranged at a proximal end of the optical waveguide (130) and is configured to detect at least a portion of the optical radiation that has returned to the proximal end of the optical waveguide via the distal end of the optical waveguide.

## Description

### FIELD OF THE INVENTION

The present disclosure relates to an optical measurement device for obtaining optical measurements from a subject sampling region. The optical measurement device may be used to obtain optical measurements such as photoplethysmography "PPG" measurements, laser speckle plethysmography "SPG" measurements, and so forth. The optical measurement device may be used to obtain optical measurements from various subject sampling regions, such as a tissue region, or a respiratory gas detection region, for example. The optical measurements may be used to evaluate a vital signs measurement for a subject, such as a blood oxygenation measurement, e.g. an SPO2 measurement; a heart rate measurement; a capnography measurement; and so forth.

### BACKGROUND OF THE INVENTION

Optical measurement devices are routinely used to obtain optical measurements from subjects. The optical measurements may be obtained from various subject sampling regions, such as a tissue region. For instance, photoplethysmography "PPG" measurements, i.e. measurements of blood volume changes, may be obtained from tissue regions in the finger, the ear, the nasal alar, the nasal septum, the forehead, and so forth. The optical measurements may then be used to evaluate a vitals signs measurement for the subject. For instance, a PPG measurement may be used to evaluate a blood oxygenation measurement, e.g. an SPO2 measurement; a heart rate measurement, and so forth. Optical measurements may also be obtained from other subject sampling regions, such as a respiratory gas detection region. The respiratory gas detection region may be within, or at least in the vicinity of, a subject's airway. Optical measurements obtained from a subject's respiratory gas detection region may likewise be used to evaluate a vitals signs measurement for the subject, including, for example a capnography measurement, i.e. measurements of the concentration of carbon dioxide in respiratory gases; a respiration rate; and so forth.

Optical measurements are typically obtained using one or more optical sources and one or more corresponding optical detectors. The optical source(s) generate optical radiation that is used to irradiate the subject sampling region. The optical detector(s) detect optical radiation that is received from the subject sampling region in response to its irradiation. Electrical signals generated by the optical detector(s) in response to the detection of the optical radiation are indicative of the optical measurements. The electrical signals are often transmitted between the subject and a console, e.g. a measurement unit, via long electrical wires.

However, optical measurement devices that employ long electrical wires to obtain optical measurements from a subject can suffer from drawbacks. For instance, they can be impacted by electrical, or magnetic fields. Such fields induce electrical interference in the electrical wires that can interfere with the electrical signals that are generated by the optical detector(s). The electrical interference can degrade the quality of the optical measurements that are made by the optical detector(s). Strong magnetic fields, such as those experienced in a magnetic resonance imaging "MRI", imaging environment, can also induce high currents in the long electrical wires. The high currents cause heating in the wires, which inturn risks harming the subject. The long electrical wires also attenuate X-ray radiation and can therefore obscure image features in imaging environments that are based on X-ray radiation.

Thus, there is a need to improve optical measurement devices that employ long electrical wires to obtain optical measurements from subjects.

### SUMMARY OF THE INVENTION

The invention is defined by the claims.

According to one aspect of the present disclosure, an optical measurement device for obtaining optical measurements from a subject sampling region, is provided. The optical measurement device includes:
- at least one optical source configured to generate optical radiation;
- an optical waveguide;
- an optical splitter or circulator; and
- at least one optical detector;

The at least one optical source is configured to couple the optical radiation into a proximal end of the optical waveguide. The optical splitter or circulator is arranged at a distal end of the optical waveguide and is configured to output at least a portion of the optical radiation for irradiating the subject sampling region, and to receive at least a portion of the outputted optical radiation that has irradiated the subject sampling region, and to couple at least a portion of the received portion back into the distal end of the optical waveguide. The at least one optical detector is arranged at a proximal end of the optical waveguide and is configured to detect at least a portion of the optical radiation that has returned to the proximal end of the optical waveguide via the distal end of the optical waveguide.

The above-described optical measurement device uses an optical waveguide to obtain optical measurements from the subject sampling region. Since the optical radiation that is transmitted along the optical waveguide is not susceptible to electrical or magnetic fields, the optical measurement device may be used in the presence of electrical and magnetic fields, e.g. MRI imaging environments, without the risk of that the fields degrade the quality of the optical measurements. The low X-ray attenuation of the optical waveguide in comparison to that of electrical conductors, also renders the optical waveguide virtually invisible in X-ray based images. Moreover, since the same optical waveguide is used to couple optical radiation both to and from the subject sampling region, the optical measurement device has improved flexibility over e.g. the use of separate optical waveguides for this purpose. The optical measurement device also includes an optical splitter, or a circulator, at a distal end of the optical waveguide. The optical splitter, or circulator, outputs optical radiation for irradiating the subject sampling region, and also couples at least a portion of the outputted optical radiation that has irradiated the subject sampling region, back into the distal end of the optical waveguide. The optical splitter, or circulator, therefore facilitates the irradiation and collection of optical radiation from the subject sampling region, via the distal end of the same optical waveguide, with a compact form factor.
Further aspects, features, and advantages of the present disclosure will become apparent from the following description of examples, which is made with reference to the accompanying drawings.

### BRIEF DESCRIPTION OF THE DRAWINGS

Fig. 1 is a schematic diagram illustrating a first example of an optical measurement device 100 for obtaining optical measurements from a subject sampling region 110, in accordance with some aspects of the present disclosure.
Fig. 2 is a schematic diagram illustrating a second example of an optical measurement device 200 for obtaining optical measurements from a subject sampling region 110, in accordance with some aspects of the present disclosure.
Fig. 3 is a schematic diagram illustrating a third example of an optical measurement device 300 for obtaining optical measurements from a subject sampling region 110, in accordance with some aspects of the present disclosure.
Fig. 4 is a schematic diagram illustrating a fourth example of an optical measurement device 400 for obtaining optical measurements from a subject sampling region 110, in accordance with some aspects of the present disclosure.
Fig. 5 is a schematic diagram illustrating a fifth example of an optical measurement device 500 for obtaining optical measurements from a subject sampling region 110, in accordance with some aspects of the present disclosure.
Fig. 6 is a schematic diagram illustrating an example of an optical measurement device 100 including an optical lens 210, 210', in accordance with some aspects of the present disclosure.
Fig. 7 is a schematic diagram illustrating an example of an optical measurement device 100 including a wavelength division multiplexer 220, in accordance with some aspects of the present disclosure.
Fig. 8 is a schematic diagram illustrating an example of an optical measurement device 100 including a first, second, and third wavelength division multiplexer 220, 220', 220", in accordance with some aspects of the present disclosure.
Fig. 9 is a schematic diagram illustrating an example of an optical measurement device 100 including a first, second, and third wavelength division multiplexer 220, 220', 220", and a second subject sampling region 110', in accordance with some aspects of the present disclosure.
Fig. 10 is a schematic diagram illustrating an example of an optical splitter 140 arranged at the distal end an optical waveguide 130, and a support 230 that supports the optical splitter in relation to a subject sampling region 110, in accordance with some aspects of the present disclosure.
Fig. 11 is a schematic diagram illustrating an example of an optical measurement device 100 including at least one processor 250, in accordance with some aspects of the present disclosure.

### DETAILED DESCRIPTION OF EMBODIMENTS

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to one optical measurement device, may be implemented in another optical measurement device, in a corresponding manner.

In the following description, reference is made to examples of an optical measurement device for obtaining optical measurements from a subject sampling region. In some examples, reference is made to optical measurements in the form of PPG measurements, and a subject sampling region in the form of a tissue region, e.g. a portion of a subject's finger, ear, nasal alar, nasal septum, forehead, and so forth. It is, however, to be appreciated that unless explicitly stated, the use of the optical measurement device is not limited to these examples. In general, the optical measurement device may be used to make a wide range of optical measurements. For instance, instead of PPG measurements, the optical measurements may be SPG measurements. Moreover, in general, the optical measurement device may be used to make optical measurements from subject sampling regions other than tissue regions. For instance, instead of being used to make optical measurements from a tissue region, the optical measurement device may be used to make optical measurements from a respiratory gas detection region. The respiratory gas detection region may be within, or at least in the vicinity of, a subject's airway.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

It is also noted that some operations that are described as being performed by the one or more processors of the system disclosed herein may be implemented using artificial intelligence techniques. Suitable techniques may include machine learning techniques, deep learning techniques, and neural networks. For instance, one or more neural networks, may be trained in a supervised, or in some cases unsupervised, manner, to implement the operations that are performed by the one or more processors.

As mentioned above, there is a need to improve optical measurement devices that employ long electrical wires to obtain optical measurements from subjects.

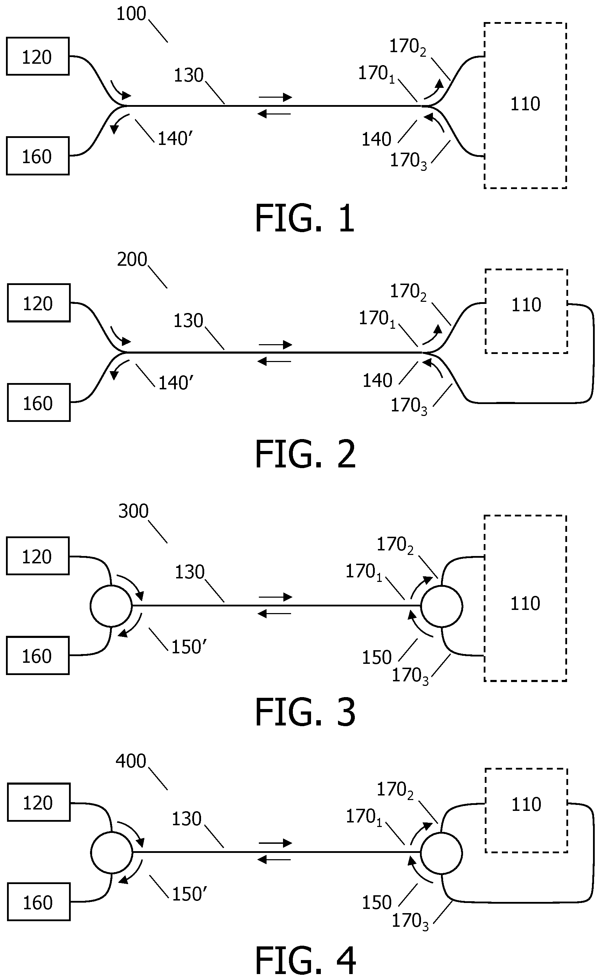

Fig. 1 - Fig. 5 illustrate various examples of optical measurement devices for obtaining optical measurements from a subject sampling region 110, in accordance with some aspects of the present disclosure. In these examples, the optical measurement device 100, 200, 300, 400, 500, includes:
- at least one optical source 120, 120'configured to generate optical radiation;
- an optical waveguide 130;
- an optical splitter 140 or circulator 150; and
- at least one optical detector 160;

The at least one optical source 120, 120' is configured to couple the optical radiation into a proximal end of the optical waveguide 130. The optical splitter 140 or circulator 140 is arranged at a distal end of the optical waveguide 130 and is configured to output at least a portion of the optical radiation for irradiating the subject sampling region 110, and to receive at least a portion of the outputted optical radiation that has irradiated the subject sampling region, and to couple at least a portion of the received portion back into the distal end of the optical waveguide 130. The at least one optical detector 160 is arranged at a proximal end of the optical waveguide 130 and is configured to detect at least a portion of the optical radiation that has returned to the proximal end of the optical waveguide via the distal end of the optical waveguide.

The above-described optical measurement device uses an optical waveguide to obtain optical measurements from the subject sampling region. Since the optical radiation that is transmitted along the optical waveguide is not susceptible to electrical or magnetic fields, the optical measurement device may be used in the presence of electrical and magnetic fields, e.g. MRI imaging environments, without the risk of that the fields degrade the quality of the optical measurements. The low X-ray attenuation of the optical waveguide in comparison to that of electrical conductors, also renders the optical waveguide virtually invisible in X-ray based images. Moreover, since the same optical waveguide is used to couple optical radiation both to and from the subject sampling region, the optical measurement device has improved flexibility over e.g. the use of separate optical waveguides for this purpose. The optical measurement device also includes an optical splitter, or a circulator, at a distal end of the optical waveguide. The optical splitter, or circulator, outputs optical radiation for irradiating the subject sampling region, and also couples at least a portion of the outputted optical radiation that has irradiated the subject sampling region, back into the distal end of the optical waveguide. The optical splitter, or circulator, therefore facilitates the irradiation and collection of optical radiation from the subject sampling region, via the distal end of the same optical waveguide, with a compact form factor.

Fig. 1 is a schematic diagram illustrating a first example of an optical measurement device 100 for obtaining optical measurements from a subject sampling region 110, in accordance with some aspects of the present disclosure. The optical measurement device 100 includes an optical source 120. The optical source 120 may be provided by a wide range of different optical emitters, including for example light emitting diodes "LEDs", e.g. Superluminescent diodes "SLDs"; lasers, e.g. Vertical-Cavity Surface-Emitting Lasers "VCSELS"; incandescent lamps, and so forth. The optical source 120 generates optical radiation. The optical source 120 may generate optical radiation within one or more wavelength intervals. For instance, the optical source may generate optical radiation within a wavelength interval that corresponds to the red portion of the optical spectrum (e.g. approximately 660 nm/ 525 nm), or within a wavelength interval that corresponds to the green portion of the optical spectrum, or within a wavelength interval that corresponds to the infrared portion of the optical spectrum (e.g. approximately 880 nm or 940 nm, respectively). Such wavelength intervals are often used to perform PPG measurements, e.g. blood oxygen saturation measurements. It is noted that whilst the optical measurement device 100 illustrated in Fig. 1 is illustrated as having a single optical source 120, the optical measurement device 100 may alternatively include multiple optical sources. Where additional optical source(s) are included, these may be provided in a similar manner.

The optical measurement device 100 illustrated in Fig. 1 also includes an optical waveguide 130. In the example illustrated in Fig. 1, there is a single optical waveguide 130, i.e. one and only one optical waveguide. The optical waveguide 130 may be provided by various types of optical waveguides, including an optical fiber waveguide. An optical fiber waveguide includes a fiber of material, such as glass (e.g. silica), or a polymer. The fiber defines an optical path for optical radiation. An optical fiber waveguide typically also includes a cladding that is formed from a material having a refractive index that value that is defined in relation to the refractive index value of the fiber so as to retain optical radiation within the fiber via total internal reflection. Instead of being provided by an optical fiber waveguide, other types of optical waveguides may serve as the optical waveguide 130, including, for example, a planar waveguide, or a strip waveguide, may be used. Planar, and strip waveguides, may likewise be formed from various materials, e.g. silicon-based materials, polymers, and so forth.

The optical measurement device 100 illustrated in Fig. 1 also includes an optical splitter 140. The optical splitter 140 is arranged at a distal end of the optical waveguide 130. The optical splitter 140 may be butt-coupled to the distal end of the optical waveguide 130, for example, e.g. by using an optical connector. An optical splitter is generally understood to be an optical component that splits optical radiation into separate paths. An optical splitter is also known as an optical combiner. Various types of optical splitters/ combiners may serve as the splitter 140, including, for example, a fiber optic splitter, a planar lightwave circuit splitter, a (polarizing) beam splitter, and a wavelength (dichroic) splitter.

The optical measurement device 100 illustrated in Fig. 1 also includes an optical detector 160. The optical detector 160 may be provided by a wide range of different types of optical detectors, including, for example photodiodes, photodetectors, and so forth. In general, the optical detector 160 is sensitive to optical radiation that is within the wavelength interval(s) of the optical radiation generated by the optical source 120.

It is noted that whilst the optical measurement device 100 illustrated in Fig. 1 is illustrated as having a single optical detector 160, it may alternatively be provided with multiple optical detectors. Where multiple optical detectors are provided, the optical detectors may be provided in a similar manner. In some examples, the multiple optical detectors may be sensitive to optical radiation within different wavelength intervals.

With continued reference to the optical measurement device 100 illustrated in Fig. 1; the optical source 120 is configured to couple optical radiation into a proximal end of the optical waveguide 130. The proximal end of the optical waveguide 130 is located towards the left-hand side of Fig. 1. In the example illustrated in Fig. 1, the optical radiation generated by the optical source 120 is coupled into the proximal end of the optical waveguide 130 by a splitter, which is referred-to herein as a second optical splitter 140'.

Instead of using the second optical splitter 140' to couple the optical radiation generated by the optical source 120 into the proximal end of the optical waveguide 130, alternative solutions are also contemplated. For instance, a circulator, or a polarizing beam splitter, may be used in this position, as described below. In one solution, the optical source 120 is a polarised optical source, and both the optical source 120 and the optical detector 160 are butt-coupled to the proximal end of the optical waveguide 130. In this solution, a polariser is configured to block optical radiation having the same polarization state as the optical radiation emitted by the optical source, from reaching the optical detector 160. This provides a compact solution without risking that the optical radiation emitted by the optical source 120 is coupled directly back into the optical detector at the proximal end of the optical waveguide 130.

As illustrated in Fig. 1, the second optical splitter 140'is arranged at a proximal end of the optical waveguide 130 and is configured to couple at least a portion of the optical radiation generated by the at least one optical source 120 into the proximal end of the optical waveguide 130. The second optical splitter 140' is also configured to couple the at least a portion of the optical radiation that has returned to the proximal end of the optical waveguide via the distal end of the optical waveguide into the at least one optical detector 160.

As illustrated in Fig. 1, the second optical splitter 140' comprises three ports. The three ports include a first port configured to receive at least a portion of the optical radiation generated by the at least one optical source 120. The three ports also include a second port configured to output at least a portion of the received optical radiation into the proximal end of the optical waveguide 130, and to receive the at least a portion of the optical radiation that has returned to the proximal end of the optical waveguide via the distal end of the optical waveguide. The three ports also include a third port configured to couple the received portion into the at least one optical detector 160. The second optical splitter 140' may be butt-coupled to the proximal end of the optical waveguide 130, for example.

It is noted that instead of using the second optical splitter 140', the coupling of optical radiation generated by the optical source 120 into the proximal end of the optical waveguide 130 may alternatively be facilitated in other ways, such as by using a circulator instead of the second optical splitter 140', or by butt coupling the optical source 120 to the proximal end of the optical waveguide 130.

Referring now to the optical splitter 140 that is arranged at the distal end of the optical waveguide 130 illustrated in Fig. 1; the optical splitter 140 is configured to output at least a portion of the optical radiation, i.e. at least a portion of the optical radiation generated by the optical source 120 that is coupled into the proximal end of the optical waveguide 130, in order to irradiate the subject sampling region 110. The distal end of the optical waveguide 130 is located towards the right-hand side of Fig. 1. The optical splitter 140 is also configured to receive at least a portion of the outputted optical radiation that has irradiated the subject sampling region, and to couple at least a portion of the received portion back into the distal end of the optical waveguide 130.

In the example illustrated in Fig. 1, the optical splitter 140 comprises three ports 170. The three ports include a first port 170₁ that is configured to receive at least a portion of the optical radiation from the distal end of the optical waveguide. The three ports also include a second port 170₂ that is configured to output at least a portion of the received optical radiation to the subject sampling region 110 for irradiating the subject sampling region. The three ports also include a third port 170₃ configured to receive at least a portion of the outputted optical radiation that has irradiated the subject sampling region 110, and to couple at least a portion of the received portion back into the distal end of the optical waveguide 130 via the first port 170₁.

In the example illustrated in Fig. 1, the irradiation of the subject sampling region 110 takes place via the second port 170₂, and the third port 170₃ receives optical radiation that has irradiated the subject sampling region 110. In this regard, the second port 170₂, and the third port 170₃ may, in-use, be directly, or indirectly, coupled to the subject sampling region 110. For instance, the second and third ports 170₂, 170₃ may be directly coupled to the subject sampling region 110 by butt-coupling the optical ports 170₂, 170₃, to the subject sampling region 110. Alternatively, the second and third ports 170₂, 170₃ may be indirectly coupled to the subject sampling region 110 by optically coupling the ports 170₂, 170₃, to the subject sampling region 110 via one or more optical components, such as an optical lens, an optical diffuser, and so forth. Such optical components may serve a wide variety of functions, such as to protect the distal ends of the ports, or to define the volume of the subject sampling region, for example.

With continued reference to the optical measurement device 100 illustrated in Fig. 1; the optical detector 160 is arranged at the proximal end of the optical waveguide 130 and is configured to detect at least a portion of the optical radiation that has returned to the proximal end of the optical waveguide via the distal end of the optical waveguide.

As mentioned above, in the example illustrated in Fig. 1, a second optical splitter 140' is arranged at the proximal end of the optical waveguide 130. In this example, the optical coupling between the optical detector 160 and the proximal end of the optical waveguide 130 is therefore facilitated by the second optical splitter 140'. If a circulator is used instead of the second optical splitter 140', then the optical coupling between the optical detector 160 and the proximal end of the optical waveguide 130 is facilitated by the circulator. Alternatively, the optical coupling may be performed in the absence of a splitter, or a circulator, at the proximal end of the optical waveguide 130, e.g. by simply butt-coupling the optical detector 160 to the proximal end of the optical waveguide 130.

It is noted that in the above description of the example illustrated in Fig. 1, the second optical splitter 140', the optical waveguide 130, and the optical splitter 140, are described as separate components wherein optical coupling between the separate components is facilitated by butt-coupling the components together. In alternative implementations, two or more of these optical components may be provided as an integral component. For instance, the second optical splitter 140' arranged at the proximal end of the optical waveguide 140, the optical waveguide 140, and the optical splitter 140 arranged at the distal end of the optical waveguide 140, may all be provided as an integral component, e.g. by fusing multiple optical fibers together.

Other types of integration of the optical components illustrated in Fig. 1 are also contemplated. For instance, two or more of the following may be provided by a photonic integrated circuit, PIC": the optical source 120, the optical detector 160, the second optical splitter 140'. A PIC is generally defined as a microchip that includes two or more photonic components that form a functioning circuit. The use of a PIC offers advantages such as miniaturization, simplified assembly, inherent component alignment, reduced cost, and so forth.

The optical measurement device 100 illustrated in Fig. 1 is configured to obtain optical reflectance measurements from the subject sampling region 110. In this regard the distal ends of the second and third ports 170₂, 170₃, of the optical splitter 140 are arranged in a side-by-side configuration with respect to the subject sampling region 110. The optical reflectance measurements may include contributions from reflected and/or scattered optical radiation. The optical measurements may be PPG measurements, or SPG measurements, for example. The subject sampling region 110 may be a tissue region, for example. The tissue region may be a portion of the subject's finger, ear, nasal alar, nasal septum, forehead, and so forth. The optical measurement device 100 illustrated in Fig. 1 may alternatively be used to obtain an optical scattering measurement, or an optical fluorescence measurement, or a combination of such measurements.

Further examples of optical measurement devices for obtaining optical measurements from a subject sampling region are illustrated in Fig. 2 - Fig. 5. The examples illustrated in these figures have features in common with the example described above with reference to Fig. 1. Features that provide the same function are labelled in the same manner, and a separate description of these features is omitted for brevity.

Fig. 2 is a schematic diagram illustrating a second example of an optical measurement device 200 for obtaining optical measurements from a subject sampling region 110, in accordance with some aspects of the present disclosure. The optical measurement device 200 illustrated in Fig. 2 differs from the optical measurement device 100 illustrated in Fig. 1 in that it is configured to obtain optical transmission measurements, rather than optical reflectance measurements, from the subject sampling region 110. In this regard, the distal ends of the second and third ports 170₂, 170₃, of the optical splitter 140 are arranged in an opposing configuration with respect to the subject sampling region 110. The optical measurement device 200 is otherwise the same as the optical measurement device 100. The optical measurement device 200 illustrated in Fig. 2 may alternatively be used to obtain an optical absorption measurement, or an optical scattering measurement, or an optical fluorescence measurement, or a combination of such measurements.

Fig. 3 is a schematic diagram illustrating a third example of an optical measurement device 300 for obtaining optical measurements from a subject sampling region 110, in accordance with some aspects of the present disclosure. The optical measurement device 300 illustrated in Fig. 3 differs from the optical measurement device 100 illustrated in Fig. 1 in that an optical circulator 150 is used instead of the optical splitter 140 at the distal end of the optical waveguide 130. A second optical circulator 150'is also used instead of the second optical splitter 140'at the proximal end of the optical waveguide 130. As with the optical measurement device 100 illustrated in Fig. 1, the optical measurement device 300 illustrated in Fig. 3 is configured to obtain optical transmission measurements from the subject sampling region 110.

An optical circulator is an optical component having three or more ports and is designed such that optical radiation entering one of its ports is emitted by the subsequent port. The direction in which optical radiation passes, or "circulates", between the courts is typically indicated with an arrow. With reference to the example illustrated in Fig. 3, optical radiation entering the first port 170₁ is emitted by the second port 170₂. Similarly, optical radiation entering the third port 170₃ is emitted by the first port 170₁. Optical radiation is therefore conveyed between the three ports in a similar manner to that described above with reference to Fig. 1. In other words, the optical circulator 150 comprises three ports 170. The three ports include a first port 170₁ that is configured to receive at least a portion of the optical radiation from the distal end of the optical waveguide. The three ports also include a second port 170₂ that is configured to output at least a portion of the received optical radiation to the subject sampling region 110 for irradiating the subject sampling region. The three ports also include a third port 170₃ configured to receive at least a portion of the outputted optical radiation that has irradiated the subject sampling region 110, and to couple at least a portion of the received portion back into the distal end of the optical waveguide 130 via the first port 170₁.

The optical circulators 150, 150' illustrated in Fig. 3 may be provided in various forms; including for example as a fiber optic circulator, or as a waveguide optical circulator. As compared to the optical splitters illustrated in Fig. 1, the optical circulators in the example illustrated in Fig. 3, may provide improved optical isolation between the different ports. The second optical circulator 150' may reduce the opportunity for optical signals to be fed-back into the optical source.

Fig. 4 is a schematic diagram illustrating a fourth example of an optical measurement device 400 for obtaining optical measurements from a subject sampling region 110, in accordance with some aspects of the present disclosure. The optical measurement device 400 illustrated in Fig. 4 differs from the optical measurement device 300 illustrated in Fig. 3 in that it is configured to obtain optical transmission measurements, rather than optical reflectance measurements, from the subject sampling region 110. In this regard, the distal ends of the second and third ports 170₂, 170₃, of the optical circulator 150 are arranged in an opposing configuration with respect to the subject sampling region 110, as in the example illustrated in Fig. 2. The optical measurement device 400 is otherwise the same as the optical measurement device 300.

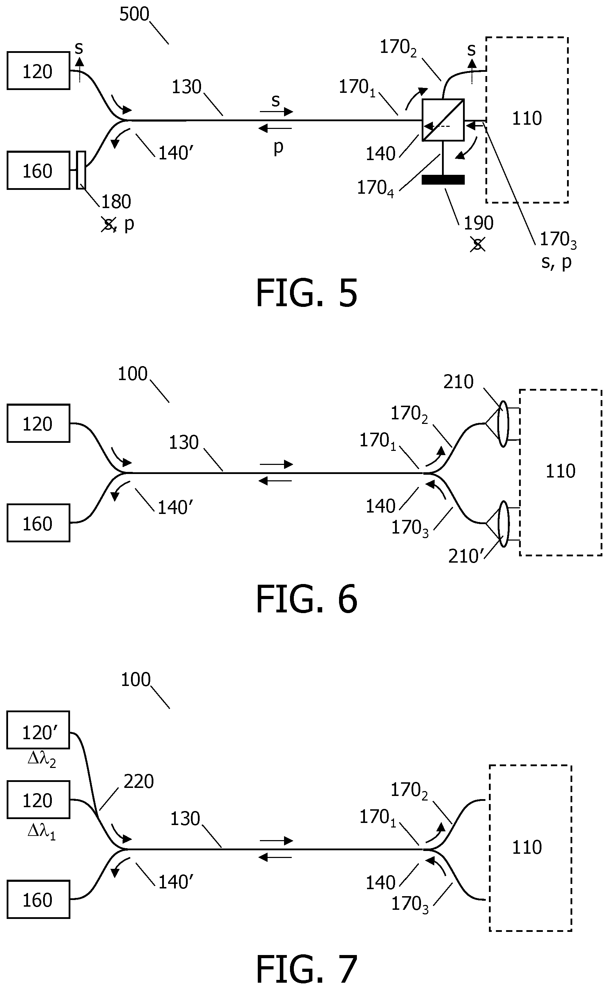

Fig. 5 is a schematic diagram illustrating a fifth example of an optical measurement device 500 for obtaining optical measurements from a subject sampling region 110, in accordance with some aspects of the present disclosure. The optical measurement device 500 illustrated in Fig. 5 differs from the optical measurement device 100 illustrated in Fig. 1 in that a polarization-maintaining optical fiber is used as the optical waveguide 130, and also in that a polarizing beamsplitter is used as the optical splitter 140. The optical radiation generated by the optical source 120 includes at least a first of two orthogonal polarization states. As illustrated in Fig. 5, the two orthogonal polarization states may be the s-, and the p-polarization states, and the first polarization state may be the s-polarization state, for example. In this example, the optical source 120 may be a laser that generates optical radiation with a defined polarization state, e.g. s-polarization. Alternatively the optical source 120 may generate non-polarized, or randomly polarized, optical radiation, and an optical polarizer (not shown in Fig. 5) that transmits only optical radiation having the first, e.g. s-, polarization state, may be coupled between the optical source and the second optical splitter 140'.

The optical measurement device 500 illustrated in Fig. 5 also includes a polarizer 180, and an absorber 190. The absorber 190 may be provided by an optically absorbing (e.g. black) surface, for example. In use, the optical radiation having the first (e.g. s-) polarization state that is generated by the optical source 120 is coupled into the proximal end of the polarization-maintaining optical fiber 130 by the second splitter 140'. The polarization-maintaining optical fiber 130 maintains the polarization state of this optical radiation such that the optical radiation arriving at the distal end of the polarization-maintaining optical fiber 130 also has the first (e.g. s-) polarization state. This optical radiation is received by the first port 170₁ of the polarizing beamsplitter 140, and outputted to the second port 170₂ with the same first (e.g. s-) polarization state, via which it irradiates the subject sampling region 110. Following its interaction with the subject sampling region 110, some of the outputted optical radiation that has irradiated the subject sampling region 110 is received by the third port 170₃. During its interaction with the subject sampling region 110, the polarization state of the optical radiation may change, e.g. as a result of optical scattering. Optical scattering may occur in a tissue region, for example. Consequently the optical radiation that is received by the third port 170₃ may have a polarization state that is different to the first (e.g. s-) polarization state of the outputted optical radiation. For example, this received optical radiation may have a random polarization. In other words, the optical radiation may have contributions from both s- and p-polarization states. In the example illustrated in Fig. 5, the received optical radiation therefore has contributions from both the first and second polarization states, i.e. s- and p-polarization.

The third port 170₃ of the polarizing beam splitter 140 couples the received optical radiation that has irradiated the subject sampling region 110 and which has only the second polarization state, p, back into the distal end of the optical waveguide 130 via the first port 170₁, as illustrated in Fig. 5. The optical radiation having the second polarization state, p, is then returned to the proximal end of the polarization-maintaining optical fiber 130. This optical radiation then passes through the polarizer 180 and is detected by the optical detector 160. The polarizer 180 is configured to transmit optical radiation having the second polarization state, p.

The polarizing beamsplitter 140 also includes a fourth port 170₄. The absorber 190 is optically coupled to the fourth port 170₄. The fourth port 170₄ emits, to the absorber 190, the optical radiation that is received by the third port 170₃ that has the first polarization state, s, such that the optical radiation having the first polarization state, s, is absorbed by the absorber 190. As a result, optical radiation having the first polarization state, s, that has interacted with the subject sampling region 110 is prevented from being coupled back into the distal and of the polarization-maintaining optical fiber 130.

In practice, it has been found that some of the optical radiation having the first polarization state, s, that is generated by the optical source 120, is reflected back into the distal end of the polarization-maintaining optical fiber 130 by the polarizing beamsplitter 140. This may due to optical reflection at the optical interface between the polarization-maintaining optical fiber 130 and the polarizing beamsplitter 140. In order to prevent that this optical radiation is detected by the optical detector 160, and therefore confounds the optical measurements of the subject sampling region 110, the polarizer 180 is arranged at the proximal end of the optical waveguide 130 between the detector 160 and the polarization-maintaining optical fiber 130. As mentioned above, the polarizer 180 is configured to transmit optical radiation having the second polarization state, p. Thus, the polarizer 180 prevents, from reaching the optical detector 160, optical radiation generated by the at least one optical source 120 having the first polarization state, s, that is returned to the proximal end of the optical waveguide 130 via the polarizing beam splitter 140. This improves the reliability of optical measurements that are made using the optical measurement device 500.

It is noted that in the example described above with reference to Fig. 5, instead of coupling an absorber 190 to the fourth port 170₄, an optical reflector may instead be coupled to the fourth port 170₄. The optical reflector may be a mirror, or a scattering surface, for example. In this position, the optical reflector couples back into the polarizing beamsplitter 140, and consequently back into the subject sampling region 110, the optical radiation that is received by the third port 170₃ that has the first polarization state, s. The optical radiation having the first polarization state, s, that is returned to the subject sampling region 110, then interacts for a second time with the subject sampling region 110. After this optical radiation has interacted with the subject sampling region 110, some of it will be received by the third optical port 170₃. If the subject sampling region 110 has scattered the optical radiation, e.g. if the subject sampling region 110 is a tissue region, some of this optical radiation will have the second polarization state, p, and will consequently be coupled to the first port 170₁ of the polarizing beamsplitter 140, following which it may be detected by the optical detector 160. Thus, if an optical reflector is instead coupled to the fourth port 170₄, the optical reflector has the effect or "recycling" optical radiation that would otherwise simply be absorbed by the absorber 190. This improves the efficiency of the optical measurement device 100.

It is also noted that instead of using the second optical splitter 140' and the polarizer 180 at the proximal end of the optical waveguide 130 (as illustrated in Fig. 3), a polarizing beam splitter that is similar to the polarizing beam splitter 140 may alternatively be included in this position.

One or more further features may be included in the optical measurement devices illustrated in Fig. 1 - Fig. 5. For instance, in one example, the optical measurement devices illustrated in these examples are provided with at least one optical lens 210, 210'. The at least one optical lens is configured to couple the optical radiation between the subject sampling region 110 and the optical splitter 140 or circulator 150. This example is illustrated in Fig. 6, which is a schematic diagram illustrating an example of an optical measurement device 100 including an optical lens 210, 210', in accordance with some aspects of the present disclosure. The optical measurement device 100 illustrated in Fig. 6 differs from the optical measurement device 100 illustrated in Fig. 1 in that it additionally includes an optical lens 210 in the optical path between the second port 170₂ of the optical splitter 140 and the subject sampling region 110, and a further optical lens 210' in the optical path between the subject sampling region 110 and the third port 170₃ of the optical splitter 140. The optical measurement device 100 illustrated in Fig. 6 otherwise corresponds to the optical measurement device 100 illustrated in Fig. 1. A description of the corresponding features is omitted for brevity. In other examples, only one of the two optical lenses 210, 210', illustrated in Fig. 6 may be included. The optical lenses 210, 210', improve the optical coupling between the optical splitter 140 and the subject sampling region 110. For instance, the optical lenses may define the volume of the subject sampling region. This in turn improves the reliability of the optical measurements that are obtained using the optical measurement device 100. The optical lenses 210, 210', may be provided by various types of optical lenses, including convex lenses, diffractive optical elements, metalenses, and so forth. It is noted that the optical lenses 210, 210', may be included in the optical measurement devices illustrated in Fig. 2 - Fig. 4 in a similar manner.

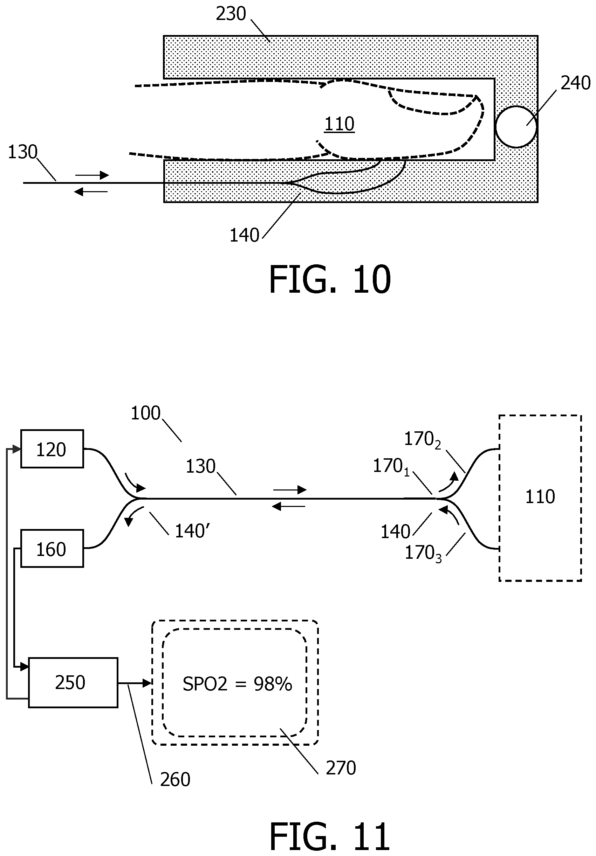

In another example, the optical measurement devices illustrated in Fig. 1 - Fig. 5 are provided with a support. The support 230 is configured to support the optical splitter 140 or circulator 150 in relation to the subject sampling region 110. Fig. 10 is a schematic diagram illustrating an example of an optical splitter 140 arranged at the distal end an optical waveguide 130, and a support 230 that supports the optical splitter in relation to a subject sampling region 110, in accordance with some aspects of the present disclosure. In the example illustrated in Fig. 10, the support 230 is configured to support a optical splitter 140 in relation to a finger 110. In this example the support 230 is provided in the form of a clip with a hinge 240 that, in-use, applies pressure to the finger to provide reliable optical coupling between the distal ends of the optical splitter 140 and the finger 110. It is noted that the support 230 may alternatively be provided in other forms, such as in the form of a sleeve for receiving a finger. Instead of supporting the splitter 140 illustrated in Fig. 1, the support 230 may alternatively support the optical splitter 140 illustrated in Fig. 2, or it may support the optical circulators 150 illustrated in Fig. 3 and Fig. 4. The support may alternatively support the optical splitter 140/ optical circulator 150 in relation to other subject sampling regions 110 than a finger; including tissue regions such as a subject's nose (e.g. nasal alar, nasal septum), ear, forehead, and so forth; and including respiratory gas detection regions such as within, or at least in the vicinity of, a subject's airway, e.g. in the form of a probe, or a mask.

In other examples, the optical measurement devices illustrated in Fig. 1 - Fig. 5 are provided with one or more wavelength division multiplexers "WDM"s. These examples are described below with reference to Fig. 7 - Fig. 9. These examples are described as adaptations of example illustrated in Fig. 1. However, these examples may alternatively be implemented as adaptations to the examples illustrated in Fig. 2 - Fig. 6.

Fig. 7 is a schematic diagram illustrating an example of an optical measurement device 100 including a wavelength division multiplexer 220, in accordance with some aspects of the present disclosure. The optical measurement device 100 illustrated in Fig. 7 differs from the optical measurement device 100 illustrated in Fig. 1 in that it includes a second optical source 120' in addition to the (first) optical source 120 illustrated in Fig. 1. The optical measurement device 100 illustrated in Fig. 7 also includes a wavelength WDM 220. A WDM, also known as a wavelength combiner, or wavelength splitters, facilitates the combining of beams of optical radiation having different wavelength ranges, into a single beam. Conversely a WDM facilitates the separation of a single beam of radiation having different wavelength ranges into different beams according to the different wavelength ranges.

In the example illustrated in Fig. 7, the optical measurement device 100 includes the wavelength division multiplexer, WDM, 220, and a first optical source 120 configured to generate optical radiation over a first wavelength range Δλ₁, and a second optical source 120' configured to generate optical radiation over a second wavelength range Δλ₂. The WDM 220 is configured to combine the optical radiation generated by the first optical source 120 with the optical radiation generated by the second optical source 120' and to couple the combined optical radiation into the proximal end of the optical waveguide 130.

Advantageously, the optical measurement device 100 illustrated in Fig. 7 may be used to obtain optical measurements corresponding to the first and second wavelength ranges, Δλ₁, Δλ₂*.* For example, the first and second optical sources may be activated simultaneously in order to obtain combined optical measurements corresponding to a combination of the first and second wavelength ranges, Δλ₁, Δλ₂*.* Alternatively, the first and second optical sources may be activated sequentially in order to obtain separate optical measurements corresponding to the first and second wavelength ranges, Δλ₁, Δλ₂. The sequential operation of the optical sources may be referred-to as time-division multiplexing "TDM". By way of an example, the optical measurement device 100 illustrated in Fig. 7, may be used to obtain from a tissue region, separate PPG measurements corresponding to each of red, and infrared, wavelength ranges. The optical measurements corresponding to the red, and infrared, wavelength ranges may then be used to evaluate a vital signs measurement, e.g. a blood oxygen saturation value, for the tissue region, as described in more detail below.

In the example illustrated in Fig. 7, the WDM 220 may be provided in various ways, including for example by a butt coupling arrangement, a prism, a diffraction grating, a fiber optic WDM, or a planar lightwave circuit WDM. The butt coupling arrangement may include butt coupling the first and second optical sources 120, 120' to the second optical splitter 140', for example.

In another example, the optical measurement device 100 illustrated in Fig. 7, is provided with a further WDM and a further optical detector (not illustrated in Fig. 7). The further WDM is coupled to the second optical splitter 140' and configured to separate the optical radiation the optical radiation that has returned to the proximal end of the optical waveguide via the distal end of the optical waveguide, into separate beams according to the different wavelength ranges Δλ₁, Δλ₂. The optical detector 160, and the further optical detector, are coupled to the further WDM so as to detect optical radiation within the wavelength ranges Δλ₁, and Δλ₂, respectively. In this example, optical radiation within both the first and second wavelength ranges, Δλ₁, Δλ₂, may be detected simultaneously, which avoids the need to operate the optical sources in a TDM manner.

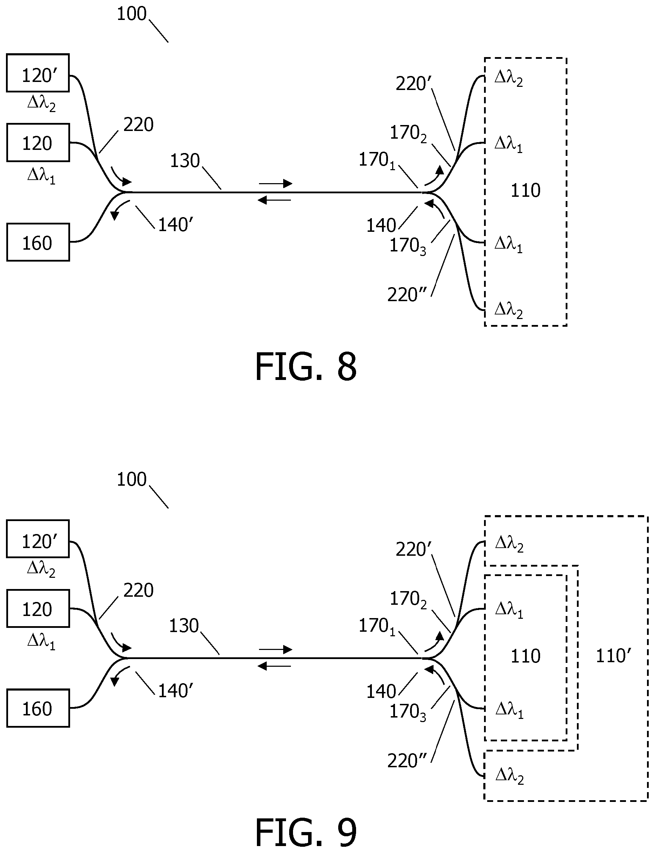

Fig. 8 is a schematic diagram illustrating an example of an optical measurement device 100 including a first, second, and third wavelength division multiplexer 220, 220', 220", in accordance with some aspects of the present disclosure. The optical measurement device 100 illustrated in Fig. 8 differs from the optical measurement device 100 illustrated in Fig. 7 in that it also includes a second WDM 220', and a third WDM 220". The first WDM 220 combines the optical radiation generated by the first optical source 120 with the optical radiation generated by the second optical source 120' and couples the combined optical radiation into the proximal end of the optical waveguide 130, as described above in relation to Fig. 7. The second WDM 220' is arranged at a distal end of the optical waveguide 130 and separates the combined optical radiation into separate irradiation beams according to the different wavelength ranges Δλ₁, Δλ₂. The irradiation beams include a first irradiation beam for irradiating the subject sampling region 110, and a second irradiation beam for irradiating the subject sampling region 110. The third WDM 220" is arranged at a distal end of the optical waveguide 130 and combines portions of the irradiation beams corresponding to the different wavelength ranges Δλ₁, Δλ₂ that are received from the subject sampling region 110 in order to provide the at least a portion of the outputted optical radiation that has irradiated the one or more subject sampling regions 110.

The optical properties of some subject sampling regions are known to be wavelength-dependent. For instance, the optical scattering of tissue regions is known to be strongly wavelength-dependent. Optical scattering can therefore impact the volume of the subject sampling region 110 from which optical measurements are obtained. Consequently, if a single optical waveguide, e.g. optical fiber, is used to irradiate a subject sampling region with optical radiation from different wavelength ranges, different portions of the subject sampling region may be sampled by the different wavelength ranges. In the optical measurement device 100 illustrated in Fig. 8, the optical irradiation is delivered to the subject sampling region 110 from different beams according to the first and second wavelength ranges, Δλ₁, Δλ₂. Likewise, optical radiation is received from the subject sampling region 110 separately according to the first and second wavelength ranges, Δλ₁, Δλ₂. This distinction between the first and second wavelength ranges, Δλ₁, Δλ₂, facilitates control over the volume of the subject sampling region 110 from which optical measurements are obtained. For instance, the different beams may be provided with different beam shapes, or delivered at different angles, in order to better match the portions of the subject sampling region that are sampled. Thus, the optical measurement device 100 illustrated in Fig. 8 may be used to ensure that optical measurements are obtained from same, or at least a similar, subject sampling region 110.

In this example, the second, and third, wavelength division multiplexers 220, 220', 220", may be provided in a similar manner to that described above in relation to Fig. 7.

Fig. 9 is a schematic diagram illustrating an example of an optical measurement device 100 including a first, second, and third wavelength division multiplexer 220, 220', 220", and a second subject sampling region 110', in accordance with some aspects of the present disclosure. The optical measurement device 100 illustrated in Fig. 9 differs from the optical measurement device 100 illustrated in Fig. 8 in that it is used to obtain optical measurements from two different subject sampling regions, i.e. the subject sampling region 110, and also a second subject sampling region 110'. By way of some examples, the subject sampling regions 110, 110' may correspond to different tissue regions, or to different respiratory gas detection regions, or to a tissue region and a respiratory gas detection region. In one example, the subject sampling region 110 is a tissue region, and the second subject sampling region 110' is a respiratory gas detection region. In this example, the optical measurement device may be used to acquire both PPG measurements, and also capnography measurements, from a subject in the presence of electrical and magnetic fields, e.g. MRI imaging environments, without the risk of that the fields degrade the quality of the optical measurements.

In another example, any of the optical measurement devices described above may be provided with one or more processors 250. The one or more processors 250 are configured to:
- receive optical measurements generated by the at least one optical detector 160 in response to an irradiation of the subject sampling region 110 with the optical radiation generated by the at least one optical source 120, 120'; and
- evaluate at least one vital signs measurement 260 based on the optical measurements.

Thus example is described with reference to Fig. 11, which is a schematic diagram illustrating an example of an optical measurement device 100 including at least one processor 250, in accordance with some aspects of the present disclosure. The example optical measurement device 100 illustrated in Fig. 11 corresponds to the example illustrated in Fig. 1, and a description of the corresponding features is omitted for brevity. As illustrated in Fig. 11, the optical measurement device 100 is in communication with one or more processors 250. The processor(s) control the optical source 120 to generate optical radiation. The processor(s) also receive optical measurements for the subject sampling region in the form of electrical signals generated by the optical detector 160 in response to the irradiation of the subject sampling region with the optical radiation generated by the optical source. In this example, the at least one vital signs measurement 260 may include at least one of: a blood oxygenation measurement, a heart rate measurement, a respiration rate measurement, and a capnography measurement.

Blood oxygen saturation measurements are typically derived from pulsed PPG measurements that are obtained over red, and infrared, wavelength intervals (e.g. approximately 660 nm and approximately 880 nm or 940 nm, respectively). Blood oxygen saturation measurements are typically evaluated using the "ratio of ratios" technique. This metric quantifies the difference in optical absorption in tissue between oxygen-bound and oxygen-unbound haemoglobin at red versus infrared wavelengths. A measurement of blood oxygen saturation may be referred-to as an "SpO₂ level. The "ratio of ratios" technique involves evaluating the so-called R-value from the PPG measurements in accordance with Equation 1. The R-value is then be converted into a blood oxygenation value using a calibration curve. ${R}_{value} = \frac{\frac{{Red}_{peak - peak}}{{Red}_{DC}}}{\frac{{IR}_{peak - peak}}{{IR}_{DC}}}$ Equation 1

In Equation 1, *Redₚₑₐₖ₋ₚₑₐₖ* is the peak - peak value of the PPG measurement for the red optical wavelength range, and *Red_{DC}* is the DC value of the PPG measurement for the red optical wavelength range. Similarly, *IRₚₑₐₖ₋ₚₑₐₖ* is the peak - peak value of the PPG measurement for the infrared optical wavelength range, and *IR_{DC}* is the DC value of the PPG measurement for infrared optical wavelength range.

A heart rate measurement may be evaluated from PPG measurements that are obtained from various tissue regions. A subject's heart rate is the dominant signal modulating a PPG measurement and may therefore be obtained by performing a frequency analysis on a PPG measurement. A subject's respiration rate measurement may be obtained from PPG measurements obtained from various tissue regions, and also from optical measurements obtained from a respiratory gas detection region. In the former case, a subject's respiration rate is modulated onto the subject's PPG measurement and may therefore be obtained by performing a frequency analysis on a PPG measurement. In the latter case, a subject's respiration rate may be obtained by monitoring an optical property (e.g. transmission, scattering, or reflectance) in a subject's respiratory gas detection region, such as within, or at least in the vicinity of, a subject's airway, over an optical wavelength range that is sensitive to changes in respiratory gas composition (e.g. oxygen, or carbon dioxide, or water vapour content).

A subject's capnography measurement may similarly be obtained by monitoring an optical property (e.g. transmission, scattering, or reflectance) in a subject's respiratory gas detection region, such as within, or at least in the vicinity of, a subject's airway, over an optical wavelength range that is sensitive to carbon dioxide.

It is noted that in addition to the one or more processors 250 illustrated in Fig. 11, in this example, the optical measurement device 100 may also include a user input device (not illustrated in Fig. 11) for receiving user input in relation to the operations performed by the processor(s) 250, such as a touchscreen, a keypad, a keyboard, a mouse and so forth. The optical measurement device 100 may also be provided with a display 270 for displaying various data associated with the operations that are performed by the processor(s) 250. The one or more processors 250 may be in communication with the display 270. The display may be configured to display information corresponding to the optical measurements and/or the value of a vital signs measurement, for example.

The above examples are to be understood as illustrative of the present disclosure, and not restrictive. Further examples are also contemplated. For instance, features described in relation to one optical measurement device, may be implemented in another optical measurement device, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. An optical measurement device (100, 200, 300, 400, 500) for obtaining optical measurements from a subject sampling region (110), the optical measurement device comprising:
at least one optical source (120, 120') configured to generate optical radiation;
an optical waveguide (130);
an optical splitter (140) or circulator (150); and
at least one optical detector (160);
wherein the at least one optical source (120, 120') is configured to couple the optical radiation into a proximal end of the optical waveguide (130);
wherein the optical splitter (140) or circulator (140) is arranged at a distal end of the optical waveguide (130) and is configured to output at least a portion of the optical radiation for irradiating the subject sampling region (110), and to receive at least a portion of the outputted optical radiation that has irradiated the subject sampling region, and to couple at least a portion of the received portion back into the distal end of the optical waveguide (130); and
wherein the at least one optical detector (160) is arranged at a proximal end of the optical waveguide (130) and is configured to detect at least a portion of the optical radiation that has returned to the proximal end of the optical waveguide via the distal end of the optical waveguide.

2. The optical measurement device according to claim 1, wherein the optical splitter (140) or circulator (150) comprises at least three ports (170), the at least three ports comprising:
a first port (170₁) configured to receive at least a portion of the optical radiation from the distal end of the optical waveguide;
a second port (170₂) configured to output at least a portion of the received optical radiation to the subject sampling region (110) for irradiating the subject sampling region; and
a third port (170₃) configured to receive at least a portion of the outputted optical radiation that has irradiated the subject sampling region (110), and to couple at least a portion of the received portion back into the distal end of the optical waveguide (130) via the first port (170₁).

3. The optical measurement device according to claim 2, wherein the optical radiation generated by the at least one optical source (120) comprises at least a first (s) of two orthogonal polarization states (s, p);
the optical waveguide (130) is a polarization-maintaining optical fiber; and
the optical splitter (140) is a polarizing beam splitter; and
wherein the optical measurement device further comprises a polarizer (180), and an absorber (190);
wherein the third port (170₃) of the polarizing beam splitter (140) is configured to couple the at least a portion of the received portion that has irradiated the subject sampling region (110) having only the second polarization state (p) back into the distal end of the optical waveguide (130) via the first port (170₁); and
wherein the polarizing beamsplitter (140) comprises a fourth port (170₄) configured to emit, to the absorber (190), the at least a portion of the received portion that has irradiated the subject sampling region (110) having the first polarization state (s) such that said optical radiation is absorbed by the absorber (190); and
wherein the polarizer (180) is arranged at the proximal end of the optical waveguide (130) and is configured to prevent from reaching the at least one optical detector (160), optical radiation generated by the at least one optical source (120) having the first polarization state (s) that is returned to the proximal end of the optical waveguide (130) via the polarizing beam splitter (140).

4. The optical measurement device according to claim 1 or claim 2, wherein the optical splitter is a fiber optic splitter, or a planar lightwave circuit splitter, or a beam splitter, or a dichroic splitter.

5. The optical measurement device according to any previous claim, further comprising at least one optical lens (210, 210');
wherein the at least one optical lens is configured to couple the optical radiation between the subject sampling region (110) and the optical splitter (140) or circulator (150).

6. The optical measurement device according to any previous claim, wherein the optical splitter (140) or circulator (150) is configured to obtain at least one of the following optical measurements from the subject sampling region (110):
an optical transmission measurement;
an optical reflectance measurement;
an optical scattering measurement;
an absorption measurement;
an optical fluorescence measurement;
and/or
wherein the subject sampling region (110) comprises at least one of the following: a tissue region;
a respiratory gas detection region.

7. The optical measurement device according to any previous claim, further comprising a second optical splitter (140') or circulator (150');
wherein the second optical splitter (140') or circulator (150') is arranged at a proximal end of the optical waveguide (130) and is configured to couple at least a portion of the optical radiation generated by the at least one optical source (120) into the proximal end of the optical waveguide (130), and to couple the at least a portion of the optical radiation that has returned to the proximal end of the optical waveguide via the distal end of the optical waveguide into the at least one optical detector (160).

8. The optical measurement device according to claim 7, wherein the second optical splitter (140') or circulator (150') comprises at least three ports, the at least three ports comprising:
a first port configured to receive at least a portion of the optical radiation generated by the at least one optical source (120);
a second port configured to output at least a portion of the received optical radiation into the proximal end of the optical waveguide (130), and to receive the at least a portion of the optical radiation that has returned to the proximal end of the optical waveguide via the distal end of the optical waveguide; and
a third port configured to couple the received portion into the at least one optical detector (160).

9. The optical measurement device according to any previous claim, further comprising at least one wavelength division multiplexer, WDM, (220) and wherein the at least one optical source comprises a first optical source (120) configured to generate optical radiation over a first wavelength range (Δλ₁), and a second optical source (120') configured to generate optical radiation over a second wavelength range (Δλ₂); and
wherein the at least one WDM (220) is configured to combine the optical radiation generated by the first optical source (120) with the optical radiation generated by the second optical source (120') and to couple the combined optical radiation into the proximal end of the optical waveguide (130).

10. The optical measurement device according to claim 9, wherein the at least one WDM, comprises a first WDM (220), a second WDM (220'), and a third WDM (220"); and
wherein the first WDM (220) is configured to combine the optical radiation generated by the first optical source (120) with the optical radiation generated by the second optical source (120') and to couple the combined optical radiation into the proximal end of the optical waveguide (130);
wherein the second WDM (220') is arranged at a distal end of the optical waveguide (130) and is configured to separate the combined optical radiation into separate irradiation beams according to the different wavelength ranges (Δλ₁, Δλ₂), the irradiation beams comprising a first irradiation beam for irradiating the subject sampling region (110) and a second irradiation beam for irradiating the subject sampling region (110) or a second subject sampling region (110'); and
wherein the third WDM (220") is arranged at a distal end of the optical waveguide (130) and is configured to combine portions of the irradiation beams corresponding to the different wavelength ranges (Δλ₁, Δλ₂) that are received from the one or more subject sampling regions (110, 110') to provide the at least a portion of the outputted optical radiation that has irradiated the one or more subject sampling regions (110, 110').

11. The optical measurement device according to claim 9 or claim 10, wherein the at least one WDM (220) is provided by at least one of: a butt coupling arrangement, a prism, a diffraction grating, a fiber optic WDM, a planar lightwave circuit WDM.

12. The optical measurement device according to any previous claim, wherein
two or more of the following are provided by a photonic integrated circuit: the at least one optical source (120), the at least one optical detector (160), the second optical splitter (140') or circulator (150'), the at least one WDM (220', 220") arranged at the distal end and/or proximal end of the optical waveguide (130).

13. The optical measurement device according to any previous claim, wherein the optical measurement device further comprises a support (230); and
wherein the support (230) is configured to support the optical splitter (140) or circulator (150) in relation to the subject sampling region (110).

14. The optical measurement device according to any previous claim, wherein the optical measurement device further comprises one or more processors (250), and wherein the one or more processors (250) are configured to:
receive optical measurements generated by the at least one optical detector (160) in response to an irradiation of the subject sampling region (110) with the optical radiation generated by the at least one optical source (120, 120'); and
evaluate at least one vital signs measurement (260) based on the optical measurements.

15. The optical measurement device according to claim 14, wherein at least one vital signs measurement (260) comprises at least one of: a blood oxygenation measurement, a heart rate measurement, a respiration rate measurement, a capnography measurement.
